Europäisches Patentamt

European Patent Office

Office européen des brevets

(11) Publication number: **0 187 516 B1**

# EUROPEAN PATENT SPECIFICATION

(45) Date of publication of patent specification: **06.03.91**　　(51) Int. Cl.⁵: **A61F 2/00, A61F 2/76**

(21) Application number: **85309409.2**

(22) Date of filing: **23.12.85**

(54) **Prosthetic alignment device.**

(30) Priority: **31.12.84 GB 8432794**

(43) Date of publication of application:
**16.07.86 Bulletin  86/29**

(45) Publication of the grant of the patent:
**06.03.91 Bulletin  91/10**

(84) Designated Contracting States:
**AT BE CH DE FR IT LI LU NL SE**

(56) References cited:
**DE-A- 3 422 127**

(73) Proprietor: **UNIVERSITY COLLEGE LONDON
Gower Street
London WC1E 6BT(GB)**

(72) Inventor: **Knox, William
22 Barnett Wood Lane
Leatherhead Surrey KT22 7DN(GB)**

(74) Representative: **Charlton, Peter John et al
ELKINGTON AND FIFE Beacon House 113
Kingsway
London WC2B 6PP(GB)**

## Description

The present invention relates to a prosthetic alignment device comprising at least two co-operating members having opposing curved surfaces.

Alignment devices are known which comprise a number of co-operating members having curved spherical surfaces which are movable relative to one another to adjust the alignment of a prosthesis. The co-operating members may have slots within which the central bolt of the device can travel and other may have abrasive surfaces to aid locking of the members together.

The known devices are complicated to adjust and the set alignment is difficult to determine.

According to the present invention there is provided a prosthetic alignment device comprising at least two co-operating members having opposing curved faces, characterized in that the faces include means for defining a direction of relative movement of the co-operating members and for calibrating that movement, the said means for defining and calibrating comprising a row of teeth on the said face (a) of one member and a corresponding row of indentation(s) on the said face (b) of the opposing member, the said faces being curved in the said direction of movement.

The rows of engaging teeth and indentations define the direction one member is to move relative to the other. The pitch of the teeth gives a predetermined angle of adjustment of the two co-operating members, and by counting the number of teeth or indentations visible the adjustment of the two members and consequently the alignment of the prosthesis can be immediately calculated.

The device preferably comprises three cooperating members having opposing spherically curved surfaces. The opposing faces of the first and second members have a row of teeth and a row of indentations and the opposing faces of the second and third members have a row of teeth and a row of indentations, these latter rows being perpendicular to the former rows. Thus, Posterior-Anterior-Lateral-Medial (P.A.L.M.) adjustment is provided for.

Preferably, the indentations are formed as a row of grooves extending perpendicular to the direction of relative movement and the teeth are in the form of a row of ribs again extending perpendicular to that direction. The rows of ribs and grooves form parallel-sided tracks which together define the direction of movement.

An embodiment of the present invention is described in detail below, by example only, with reference to the accompanying drawings, wherein:

Fig. 1 is partly sectional and partly elevational view of a device according to the invention fixed in a prosthesis; and

Fig. 2 is a view from below of one of the cooperating members of the device of Fig. 1.

The alignment device illustrated comprises three cooperating members 1,2 and 3 having opposing spherical faces a, b, c and d. A row of teeth, e.g. 4; on one member is engageable with a row of indentations 15,(seen best at 5) on another member.

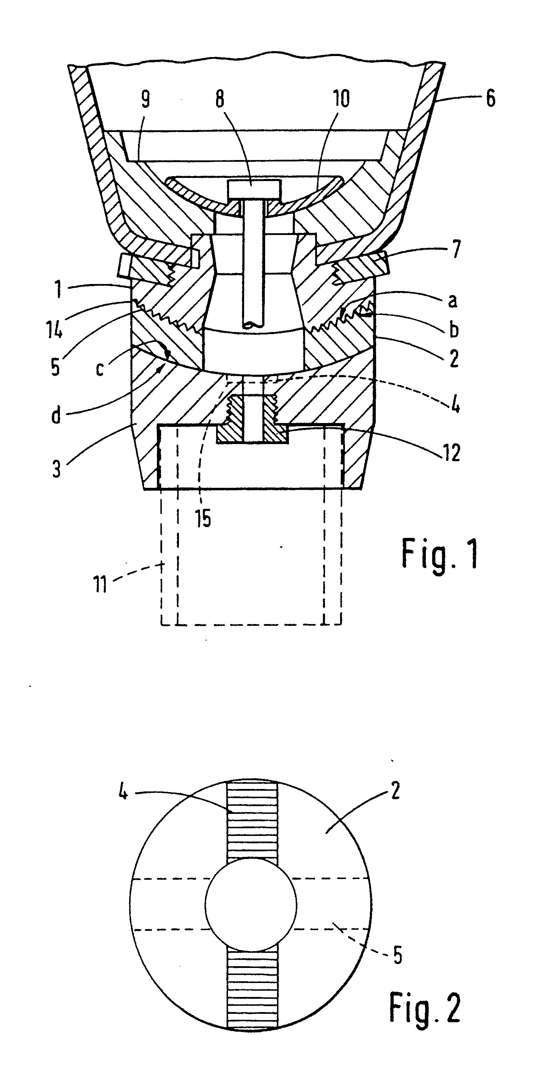

The individual parts of the device and the other prosthetic elements shown in Fig. 1 are discussed below. Locking Unit 1 - (first cooperating member) This acts as a mounting between the moulded socket 6 and the P.A.L.M. adjusting plate 2 (second cooperating member) giving both locking and prevention of turning on the centre line axis during both the alignment stage and final assembly. It has a ribbed location track 14 on its curved face a.
Jacking Ring - 7
This pushes against the outer face of the socket 6 to pretension centre bolt 8.
Spherical Cup 9
This provides the spherical movement to be accommodated by the central bolt 8 and gives an even distribution of load on the internal bearing surface of the socket 6. Spherical Washer 10 Provides a bearing surface for transmission of loads to the socket via the central bolt 8 at any pre-set angle or zero position.
P.A.L.M. Adjustment Plate - 2 (second cooperating member). This provides a ribbed location track 4 on its face C and at right angles a grooved location track 5 on its face 6 to prevent assembled component turning on axis; both tracks are provided with a series of ribs or grooves pitched at $2°$ or other convenient value to give angular setting of the device together with visual reference to the angles so set.
Spherical Shin Coupling Adaptor - 3 (third cooperating member)
This enables the assembled device to be mounted on a suitable shin component 11 that has a compatible interface, in order to transfer load to either a similar device as described above, or a fixed or flexible ankle interface as commonly used for a prosthetic foot. It includes a grooved indentation track 15 on its face d. Centre Bolt - 8
Provides clamping force to retain all components during adjustment and final assembly. The members 1 and 2 have large central apertures to accomodate the relative movement of the bolt as the members are adjusted. Clamping Nut - 12
This is fitted in the member 3 and transfers force from bolt to under face of spherical shin coupling adaptor 3. Fig. 2 shows the disc-shaped adjustment plate 2 having, on one face, a row of ribs 4 in the form of a parallel-sided track and, on the other face, a row of grooves 5 in the same track design. The row 4 is perpendicular to the row 5 to provide full P.A.L.M. adjustment.

The tracks 14 and 15 on the members 1 and 3 are of a corresponding design so the sides of the engaging tracks abut to define the direction of relative movement. The ribs and the grooves extend across the tracks, perpendicular to the direction of movement, and are of the same shape so that, of course, a rib can be considered as a groove as desired.

The row of ribs may be integrally moulded with the cooperating member or may be or separate strip fixed to the adjustment plate.

To use the invention, the device is assembled as shown in Fig. 1 with the jacking ring 7 screwed down in contact with bearing face of the locking unit item 1, together with the centre bolt 8 screwed in position with sufficient tension to hold the assembly together without rotation, but still allowing adjustment of the spherical surfaces in relationship to each other, by moving in the direction of the tracks. When a determined position in one or both planes is established by reference to visual indication, the jacking ring is unscrewed away from the location face of member 1 bringing it to bear against outer face of the prosthetic socket 6, thereby effectively tensioning the centre bolt 8 to draw all components together and locking them to prevent unintentional movement taking place. This is to enable the patient to bear his weight during both static and normal walking assessment that is conventionally used. If the adjustment is satisfactory the limb can then be removed from the patient; the reference points as visually indicated by the number of visible ribs or grooves are then noted. The jacking ring 7 is then screwed firmly down to its home position, and the centre bolt 8 is finally tightened to firmly hold the assembly together in the noted angular position to enable the device to sustain the full dynamic loads that occur during strenuous use through its service period.

As can be observed, the locking can be carried out during the alignment stage by the use of a simple tool externally to the artificial limb. This gives a distinct advantage both to the patient and the person carrying out the adjustments.

The visual indicator gives an adjustment increment of 2° steps to a maximum of 10° either side of the centre line in any P.A.L.M. plane, but the steps could be of any other convenient value.

The above device as described and illustrated would also enable similar adjustments to be made at the ankle interface of a prosthetic foot by simply inverting the assembly and using the jacking ring to bear against the foot unit in order the apply tension to lock the unit together.

The spherical alignment mounting of the invention may consist of a number of injection moulded components and stainless steel tensioning units that give all the requirements of alignment and this, together with the low cost of production and corrosion resistance, provides a considerable improvement of any previously designed alignment device and overcomes the following disadvantages of known devices:

1. Requirement of the shoe or limb to be removed before access is available to locking devices.

2. Adjustments carried out are often by adjustable screws or slots without direct angles being indicated, unless an external or remote measuring device used.

3. Many devices are extremely time consuming and difficult to adjust due to design limitations and subject to corrosion as they are manufactured from metals.

## Claims

1. A prosthetic alignment device comprising at least two co-operating members (1,2) having opposing curved faces (a,b), characterized in that the faces include means for defining a direction of relative movement of the co-operating members (1,2) and for calibrating that movement, the said means for defining and calibrating comprising a row of teeth (14) on the said face (a) of one member (1) and a corresponding row of indentations (5) on the said face (b) of the opposing member (2), and the said faces being curved in the said direction of movement.

2. A device according to claim 1 characterized by three (1,2,3) co-operating members having opposing spherically curved surfaces (a,b,c), the opposing faces (a,b) of the first (1) and second (2) members having a row of teeth (14) and a row of indentations (5) and the opposing faces (c,d) of the second (2) and third members (3) having a row of teeth (4) and a row of indentations, the latter rows being perpendicular to the former rows.

3. A device according to claim 1 or 2, characterized in that the indentations (5) are formed as a row of grooves extending perpendicular to the direction of relative movement and the teeth are in the form of a row of ribs again extending perpendicular to that direction, the rows of ribs and grooves forming parallel-sided tracks which together define the direction of movement.

4. A device according to claim 3, characterized by a stump socket (6) to which the first co-operating member (1) is attached, the stump socket and the three members being connected by a central bolt (8) which is locked by means of a nut (12) in the third co-operating member (3).

5. A device according to claim 4, characterized in that the stump socket (6) includes means to provide for the movement of the central bolt (8) within the socket (6) and for the even distribution of load from the bolt (8) to the socket (6).

6. A device according to claim 4 or 5, characterized in that the first (1) and second (2) co-operating members have central apertures which accommodate the movement of the bolt (8) as the members are moved relative to one another.


**Revendications**

1. Dispositif d'alignement de prothèse, comportant au moins deux éléments coopérants (1,2) présentant des faces incurvées (a,b) situées en face l'une de l'autre, caractérisé par le fait que les faces comportent des moyens pour définir une direction du mouvement relatif des éléments coopérants (1,2) et pour graduer ce mouvement, lesdits moyens prévus pour définir et graduer comportant une rangée de dents (14) sur ladite face (a) de l'un des éléments (1) et une rangée correspondante d'indentations (5) sur ladite face (b) de l'élément (2) situé en face, et lesdites faces étant incurvées dans ladite direction du mouvement.

2. Dispositif selon la revendication 1, caractérisé par trois éléments coopérants (1,2,3) présentant des surfaces incurvées sphériquement (a,b,c) situées en face l'une de l'autre, les faces (a,b), situées en face l'une de l'autre, du premier (1) et du second (2) éléments présentant une rangée de dents (14) et une rangée d'indentations (5) et les faces (c,d), situées en face l'une de l'autre, du second (2) et du troisième (3) éléments présentant une rangée de dents (4) et une rangée d'indentations, les dernières rangées étant perpendiculaires aux premières.

3. Dispositif selon la revendication 1 ou 2, caractérisé par le fait que les indentations (5) ont la forme d'une rangée de rainures s'étendant perpendiculairement à la direction du mouvement relatif et par le fait que les dents ont la forme d'une rangée de nervures s'étendant à nouveau perpendiculairement à cette direction, les rangées de nervures et de rainures formant des pistes à bords parallèles qui définissent ensemble la direction du mouvement.

4. Dispositif selon la revendication 3, caractérisé par un réceptacle en tronc de cône (6) auquel est fixé le premier élément coopérant (1), le réceptacle en tronc de cône et les trois éléments étant reliés par un boulon central (8) qui est bloqué par un écrou (12) dans le troisième élément coopérant (3).

5. Dispositif selon la revendication 4, caractérisé par le fait que le réceptacle en tronc de cône (6) comporte des moyens pour permettre le mouvement du boulon central (8) à l'intérieur du réceptacle (6) et pour assurer la distribution régulière de la charge entre le boulon (8) et le réceptacle (6).

6. Dispositif selon la revendication 4 ou 5, caractérisé par le fait que le premier (1) et le second (2) éléments coopérants présentent une ouverture centrale qui autorise le mouvement du boulon (8) lorsque les éléments se déplacent l'un par rapport à l'autre.


**Ansprüche**

1. Prothesenausrichtungsvorrichtung, umfassend wenigstens zwei zusammenwirkende Teile (1, 2), die gegenüberliegende gekrümmte Flächen (a, b) haben, dadurch **gekennzeichnet,** daß die Flächen Mittel zum Definieren einer Richtung der relativen Bewegung der zusammenwirkenden Teile (1, 2) und zum Kalibrieren dieser Bewegung aufweisen, wobei die Mittel zum Definieren und Kalibrieren eine Reihe von Zähnen (14) auf der Fläche von einem Teil (1) und eine entsprechende Reihe von Einschnitten (5) auf der Fläche (b) des gegenüberliegenden Teils (2) umfassen, und wobei die Flächen in der Richtung der Bewegung gekrümmt sind.

2. Vorrichtung nach Anspruch 1, **gekennzeichnet** durch drei (1, 2, 3) zusammenwirkende Teile, die gegenüberliegende sphärisch gekrümmte Oberflächen (a, b, c) haben, wobei die gegenüberliegenden Flächen (a, b) des ersten (1) und zweiten (2) Teils eine Reihe von Zähnen (14) und eine Reihe von Einschnitten (5) haben und die gegenüberliegenden Flächen (c, d) des zweiten (2) und dritten (3) Teils eine Reihe von Zähnen (4) und eine Reihe von Einschnitten haben, wobei die letzteren Reihen senkrecht zu den ersteren Reihen sind.

3. Vorrichtung nach Anspruch 1 oder 2, dadurch **gekennzeichnet,** daß die Einschnitte (5) als eine Reihe von Vertiefungen ausgebildet sind, die sich senkrecht zu der Richtung der relativen Bewegung erstrekken, und die Zähne die Form einer Reihe von Rippen haben, die sich ebenso senkrecht zu jener Richtung erstrekken, wobei die Reihen von Rippen und Vertiefungen parallelseitige Bahnen bilden, welche zusammen die Richtung der Bewegung definieren.

4. Vorrichtung nach Anspruch 3, **gekennzeichnet** durch einen Stumpfsockel (6), an dem das erste zusammenwirkende Teil (1) angebracht ist, wobei der Stumpfsockel und die drei Teile durch eine mittige Schraube (8) verbunden sind, die durch eine Mutter (12) in dem dritten zusammenwirkenden Teil (3) arretiert wird.

5. Vorrichtung nach Anspruch 4, dadurch **gekennzeichnet,** daß der Stumpfsockel (6) Mittel zum Vorsehen der Bewegung der mittigen Schraube (8) innerhalb des Sockels (6) und der gleichmäßigen Verteilung der Belastung von der Schraube (8) auf den Sockel (6) aufweist.

6. Vorrichtung nach Anspruch 4 oder 5, dadurch **gekennzeichnet,** daß das erste (1) und zweite (2) zusammenwirkende Teil eine mittige Öffnung haben, welche die Bewegung der Schraube (8) aufnimmt, wenn die Teile raltiv zueinander bewegt werden.

Fig. 1

Fig. 2